# FASCICULE DE BREVET EUROPEEN

(11) **EP 1 034 259 B1**
(45) Date de publication et mention de la délivrance du brevet: **03.05.2006**
(21) Numéro de dépôt: 98958954.4
(22) Date de dépôt: 04.12.1998
(51) Int. Cl.: C12N 15/10, C12N 1/21, C12N 9/22, C12N 15/70, C12Q 1/68

(54) **PROCEDE DE CLONAGE PAR DIGESTION MULTIPLE**
KLONIERUNGSVERFAHREN DURCH MULTIPLE VERDAUUNG
CLONING METHOD BY MULTIPLE DIGESTION

(30) Priorité: 04.12.1997 FR 9715319
(43) Date de publication de la demande: 13.09.2000
(73) Titulaire: Biomethodes S.A.R.L., 75008 Paris (FR)
(72) Inventeur: DELCOURT, Marc, F-75013 Paris (FR)
(74) Mandataire: Dossmann, Gérard
(86) Numéro de dépôt international: PCT/FR1998/002629
(87) Numéro de publication internationale: WO 1999/028451

(56) Documents cités:
- WO-A-95/04745
- US-A- 5 252 724
- JONES D.H. ET AL.: "Production of a vector to facilitate DNA mutagenesis and recombination" BIOTECHNIQUES, vol. 16, no. 4, 1994, pages 694-701, XP002076171 cité dans la demande
- DENG W.P. & NIKOLOFF J.A.: "Site-directed mutagenesis of virtually any plasmid by eliminating a unique site" ANALYTICAL BIOCHEMISTRY, vol. 200, 1992, pages 81-88, XP002096494
- GALLI ET AL.: "Mammalian genomic sequences can substitute for the SV40 AT stretch in sustaining replication of the SV40 origin of replication" FEBS LETTERS, vol. 318, no. 3, mars 1993, pages 335-340, XP002096461
- MAJUMDER K ET AL: "Recombinant enrichment by exploitation of restriction sites with interrupted palindromes: design, synthesis and incorporation of zero-background linkers in cloning and expression vectors" GENE, vol. 151, no. 1, 30 décembre 1994, page 147-151 XP004042627
- AMERSHAM LIFE SCIENCE CATALOGUE 1994 pages 164-165 XP002076172
- MANDECKI ET AL: 'A totally synthetic plasmid for general cloning , gene expression and mutagenesis in E.coli' GENE vol. 94, 1990, pages 103 - 107
- BRÜHLMANN ET AL: 'Cloning, sequence and expression of the pel gene from amycolata sp.' GENE vol. 202, 20 Novembre 1997, pages 45 - 51
- ATCHISON ET AL: 'Construction of rare restriction site ( NotI, SacII and ClaI) linking libraries and sequence-tagged site analysis of single copy clones from a human chromosome-3-specific library' GENE vol. 151, 1994, pages 325 - 328

## Description

La présente invention concerne le domaine de la biologie moléculaire et se rapporte plus particulièrement au clonage de gènes.

Le clonage de gènes est un domaine en pleine expansion qui vise notamment à associer fonctions et gènes. Ce domaine se développe principalement selon deux grands axes, d'une part la biologie moléculaire inverse, qui consiste à séquence de façon massive des banques d'ADN génomiques ou complémentaires, et d'autre part la biologie moléculaire directe, qui consiste à trouver la séquence responsable d'une activité observée, comme une activité enzymatique ou une homologie avec d'autres gènes.

La présente invention vise précisément à offrir une nouvelle méthode de clonage d'un fragment d'acide nucléique, désignée aussi ci-après DMD pour "Digestion Multiple Différentielle", qui repose sur l'utilisation systématique, combinée, et préparative des sites de restriction présents sur les inserts constituant une banque d'ADN complémentaire ou génomique.

La DMD s'applique notamment aux cas du criblage de banques d'expression et du clonage par homologie.

La présente invention trouve également des applications dans le domaine fastidieux du séquençage quand celui-ci n'a qu'une activité d'identification des inserts, ainsi que dans l'étude du polymorphisme humain, notamment dans le cadre de la recherche des prédispositions génétiques.

L'article de Galli et ses collaborateurs, (Galli, I., *et al*., (1993), Mammalian genomic sequences can substitute for the SV40 AT stretch in sustaining replication of the SV40 origin of replication, *FEBS Letters,* **318**, pp335-40) décrit une méthode de clonage d'une banque génomique en utilisant les sites de restriction *Mbo*I et *Xba*I d'un vecteur dérivé de PUC19. Cependant, aucun crible spécifique des fragments clonés n'est ensuite effectué sur les différents clones obtenus, ce qui contraint le manipulateur à des tests fastidieux.

Ce problème est résolu par le procédé de l'invention qui est un procédé universel d'isolement d'un fragment d'acide nucléique dans une banque, permettant de cloner plus facilement le fragment d'acide nucléique désiré.

On désignera ci-après par Caractéristique Enzymatique (CE) la résistance, notée "r" ou la sensibilité, notée "s", d'un fragment d'acide nucléique à une enzyme de restriction. Ce qui signifie :
- qu'un fragment qualifié "s" pour une enzyme de restriction donnée contient le site de clivage de cette enzyme, et
- qu'un fragment qualifié "r" pour une enzyme de restriction ne contient pas le site de clivage de cette enzyme.

La Caractéristique Enzymatique Multiple (CEM) est alors l'ensemble des CE obtenues pour plusieurs enzymes. On peut alors représenter la CEM d'un fragment d'ADN contenant un site Eco, un site Bam, deux sites Sca, mais pas de site Hind ni Stu, de la façon suivante: Eco^{s}Bam^{s}Sca^{s}Hind^{r}Stu^{r}.

Le procédé de l'invention propose donc d'associer au fragment, que l'on cherche à isoler au sein d'un échantillon, sa caractéristique enzymatique multiple (CEM). La recherche dudit fragment peut être réalisée par tout moyen connu de l'homme du métier, comme sa capacité à hybrider à une sonde nucléique, l'activité enzymatique de son produit protéique, l'expression d'une protéine que l'on pourra détecter, etc...

Le procédé de clonage selon l'invention est fondé sur la démonstration que quand le nombre d'enzymes est suffisamment grand, chaque insert de la banque possède une CEM originale, et qu'en conséquence, l'invention offre une stratégie simple de clonage des gènes à partir de leur CEM. Le procédé selon l'invention repose donc sur le balayage de banques d'ADN en utilisant de façon combinatoire la répartition des sites de restriction sur les inserts constituant cette banque.

Le principe du procédé selon l'invention repose également sur l'utilisation d'un vecteur de type nouveau car substantiellement dépourvu de tous les sites de clivage par des enzymes de restrictions pour n'en garder que ceux nécessaires à la construction de la banque, orientée ou non, et à son éventuel sous-clonage dans un autre vecteur. Dans une forme particulière de réalisation minimale, le vecteur contient :
- un site A nécessaire pour construire la banque, et
- deux sites B, identiques et flanquant le site A, utile pour sous-cloner le gène une fois celui-ci identifié et cloné.

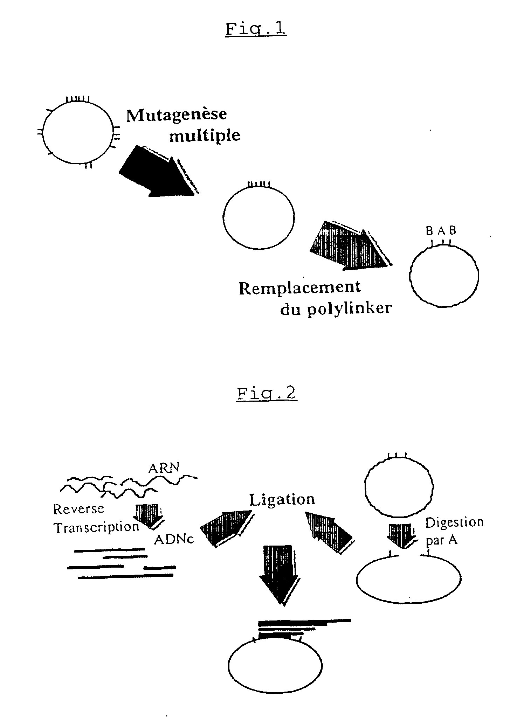

Cet ensemble pourra être désigné ci-après "trilinker", dont la fabrication schématique est donnée à la figure 1 en annexe.

Dans une autre forme particulière de réalisation, le vecteur contient :
- un site A et un site A', différents, pour construire la banque, et
- deux sites B et B', identiques ou différents, et flanquant les sites A et A', pour sous-cloner le gène une fois celui-ci identifié et clone.

Avantageusement les sites B sont des sites octonucléotidiques, de façon à minimiser le risque que des sites B soient présents dans les inserts clonés. Il sera ainsi possible de sous-cloner facilement en un seul morceau.

Environ 100 enzymes de restriction à site hexanucléotidique ont été découvertes à ce jour. 70 d'entre-elles ont un site de reconnaissance de type palyndrome continu ou discontinu.

Un vecteur de l'invention, avantageusement un plasmide, ne contient plus, mis à part ces trois sites, de sites de restriction hexa ou penta-nucléotidiques correspondant à des enzymes de restriction déjà identifiées ou qui le seront dans le futur. On entend donc par "substantiellement", que cette destruction peut être partielle en ce sens qu'elle ne concerne que certains des sites connus; seules les enzymes correpondantes seront alors utilisées dans le procédé de l'invention. Dans l'exposé de l'invention qui suit, on considère, comme indiqué précédemment que environ 50 à 70 types de sites ont été détruits.

Un vecteur de l'invention peut être construit à partir d'un plasmide déjà existant et possédant toutes les fonctions nécessaires pour permettre d'y créer et d'y manipuler une banque d'ADNC ou génomique. Il peut être utile, dans le procédé de l'invention, que la banque ne contienne plus de vecteur seul refermé sur lui-même. Il est donc avantageux que le vecteur de l'invention contienne un système qui élimine tout vecteur seul refermé sur lui-même, comme un gène suicide, un système de disruption de la proximité d'un promoteur lambda ou tout autre système connu de l'homme du métier.

La technique utilisée, dans le cadre de l'invention pour détruire tous les sites de restriction est une des techniques de mutagenèse dirigée simple ou multiple déjà décrites dans l'art antérieur, ou tout autre technique connue par l'homme du métier, comme le remplacement de segments du plasmide par oligonucléotides. On obtient ainsi un vecteur résistant à 70 enzymes de restriction, numérotées de I à LXX, et sensible à 2, désignées ci-avant A et B. L'idée de détruire simultanément la majorité des sites de restriction présents sur un plasmide a été envisagée par D. H. Jones et al. (BioTechniques 1994, 16, 4 : 694) mais dans un contexte tout différent. En effet, cet article décrit la destruction par mutagénèse multiple de 31 des 37 sites d'un très petit vecteur, de façon à créer un nouvel outil facilitant certaines manipulation de l'ADN.

Le procédé de l'invention est par ailleurs basé sur la création d'une banque d'ADN, laquelle selon l'application envisagée contient de 1 à 10⁸ et de préférence de l'ordre de 10⁵ à 4.10⁶ fragments différents de l'ordre de 0,1 kb à 5 kb chacun, et de préférence, selon les applications du procédé de l'invention, de 1 à 2 kb. Dans le mode particulier de mise en oeuvre du procédé de l'invention décrit à l'exemple 6 ci-après concernant l'étude du polymorphisme, la banque peut ne comprendre qu'un seul fragment.

Ainsi, pour l'application du procédé de l'invention à la fabrication de banques d'expression ou au clonage par homologie, il a été préparé, comme représenté schématiquement à la figure 2 en annexe, une banque d'ADNc de 10⁵ fragments différents de 1 kb chacun.

Toutefois, ce modèle constitue une approximation et est donc légèrement faux, car la taille des fragments est hétérogène. La taille moyenne des inserts étant sous-estimée et la taille de la banque étant surestimée, ce modèle a été choisi afin de disposer d'une base simple (homogénéité de la taille des fragments) et d'un système d'étude qui désavantage l'analyse (taille de la banque surestimée, taille des fragments sous-estimée) de façon à ce que le procédé de l'invention soit reproductible dans tous les cas.

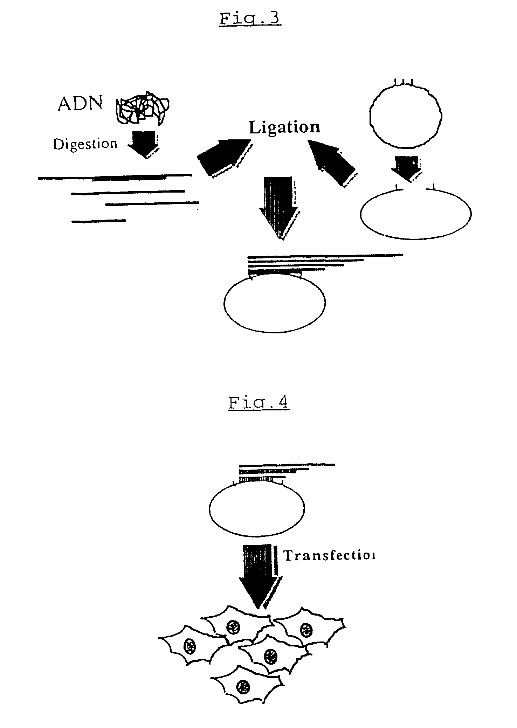

Pour l'application du procédé de l'invention au Southern Blot d'identification et à l'étude du polymorphisme, il a été préparé, comme représenté schématiquement à la figure 3 en annexe, une banque d'ADN génomique de 4.10⁶ fragments différents de 1kb chacun. Cette banque a été obtenue par l'utilisation d'une enzyme correspondant à un site de fréquence théorique 1/1024 (de type AT (ACGT) (TGCA) TA).

Tous types de banques d'ADN, tels que celles obtenus par amplification au hasard par PCR utilisant des oligonucléotides dégénérés ou non, entrent dans le cadre de la présente invention.

En conséquence, l'invention a pour objet un procédé de clonage d'un fragment d'acide nucléique comprenant les étape suivantes :
- on prépare une banque d'ADN susceptible de contenir ledit fragment,
- on crible ladite banque en utilisant de façon combinatoire au moins 10 et de préférence 50 à 70 enzymes de restriction, pour isoler par tout moyen approprié le clone contenant ledit fragment.

Dans ce procédé, la préparation de la banque d'ADN susceptible de contenir le fragment d'acide nucléique, consiste à insérer chacun des fragments d'ADN d'un échantillon dans un vecteur dépourvu de tout site de clivage par des enzymes de restriction à l'exception :
- d'un site pour la construction de la banque, et
- éventuellement de deux autres sites identiques, différents du premier ou des premiers site(s) et le(s) flanquant, utiles pour sous-cloner la séquence d'acide nucléique une fois celle-ci identifiée et clonée.

Dans une autre forme de réalisation de ce procédé, on insère chacun des fragments d'ADN d'un échantillon dans un vecteur substantiellement dépourvu de tout site de clivage par des enzymes de restriction à l'exception :
- de deux sites pour la construction de la banque, orientée, et
- éventuellement de deux autres sites identiques ou non, différents des deux premiers sites et le(s) flanquant, utiles pour sous-cloner la séquence d'acide nucléique une fois celle-ci identifiée et clonée.

Le procédé comprend plus particulièrement les étapes suivantes :
a) on prépare une banque d'ADN susceptible de contenir ledit fragment d'acide nucléique, consistant à insérer chacun des fragments d'ADN d'un échantillon dans un vecteur substantiellement dépourvu de tout site de clivage par des enzymes de restriction mais conservant :
   - un ou deux sites pour la construction de la banque, et
   - éventuellement au moins deux autres sites, et de préférence seulement deux sites, identiques ou non, et différent du ou des premiers sites et le(s) flanquant, utiles pour sous-cloner le fragment d'acide nucléique une fois celui-ci identifié et cloné.

   L'échantillon à partir duquel sont issus les fragments constituant la banque peut être toute cellule eucaryote (mammifère, plante, levure, etc...), ou tout organisme procaryote (virus, bactérie, etc...). Il peut s'agir d'ADN génomique, d'ADNc, de fragments d'amplification par PCR ou de tout autre banque d'ADN susceptible d'être préparée par l'homme du métier.
b) On réalise la digestion en parallèle de la banque par plusieurs enzymes de restriction, au moins 10 et de préférence environ 50 à 70 enzymes de restriction de façon à obtenir autant de banques mono-digérés que d'enzymes utilisées.
c) On transfecte indépendamment les banques mono-digérées dans des hôtes cellulaires appropriés de façon à obtenir des lots correspondant d'hôtes cellulaires.
d) On teste par tout moyen approprié chacun des lots obtenus à l'étape (c) pour évaluer l'intégrité de la séquence d'acide nucléique à cloner et ainsi établir sa CEM.
   En effet, si la digestion par une enzyme désignée "I" n'altère pas l'intégrité de l'insert présent dans la banque, celui-ci est considéré I^{r} au contraire, si elle l'altère, celui-ci est considéré I^{s}.
   Les étapes (a) à (d) ci-dessus permettent l'analyse du fragment à cloner selon l'invention.
   Le procédé de l'invention comprend en outre les étapes suivantes permettant de purifier le fragment à cloner :
e) On reprend la banque totale de l'étape (a) et on la digère sensiblement simultanément par les enzymes qui n'affectent pas l'intégrité du fragment à cloner. Pour lesquelles il a donc été considéré "r".
f) On isole le clone résistant contenant le fragment d'acide nucléique à cloner par tout moyen approprié et éventuellement on le sous-clone en utilisant les deux sites ménagés dans le vecteur à cet effet.
g) On séquence éventuellement le fragment d'acide nucléique à cloner.

La digestion multiple de l'étape (e) a pour effet de cliver la totalité, ou la quasi-totalité, des fragments d'ADN constituant la banque, à l'exception du fragment à cloner.

L'isolement du clone résistant contenant le fragment d'acide nucléique à cloner de l'étape (f) peut être réalisé par transformation de la banque multi-digérée dans des bactéries compétentes, ou par PCR en utilisant des oligonucléotides amorces flanquant le site de clonage de la banque.

Eventuellement, par sécurité on peut effectuer des étapes de vérification entre les étape (a) et (b) d'une part, et (e) et (f) d'autre part consistant :
a') A vérifier la présence du fragment d'acide nucléique à cloner dans la banque en transfectant dans un hôte cellulaire qui n'a pas ledit fragment et en testant par tout moyen approprié la présence du fragment dans ledit hôte. Avantageusement, on utilise dans cette étape (a') des cellules COS, classiquement mise en oeuvre pour les transfections.
e') A transformer la banque multi-digérée de l'étape (e) dans des hôtes compétents de façon à vérifier la nature des fragments clonés. Par exemple, cette étape consiste à étaler sur petri et vérifier par minipréparations d'ADN plasmidique (miniprep) que les inserts sont bien sensibles aux enzymes notées "s" lors de l'établissement de la CEM.

Avantageusement, la banque d'ADN préparée à l'étape (a) contient de 1 à 10⁸ et de préférence de l'ordre de 10⁵ à 4.10⁶ fragments différents de l'ordre de 0,1 kb à 5 kb chacun, et de préférence de l'ordre de 1 à 2 kb.

Dans le procédé de l'invention, on préfère que les deux sites de sous-clonage soient des sites octonucléotidiques, de façon à minimiser le risque que des sites B soient présents dans les inserts clonés.

Dans un mode de réalisation tout particulier du procédé de l'invention, la banque ne contient plus de vecteur seul refermé sur lui-même. Il est donc avantageux que le vecteur de l'invention contienne un système qui élimine tout vecteur seul refermé sur lui-même, comme un gène suicide, un système de disruption de la proximité d'un promoteur lambda ou tout autre système connu de l'homme du métier.

Les tests réalisés aux étapes (d) et (a') pour vérifier l'intégrité de la séquence d'acide nucléique à cloner peuvent être tout moyen de mise en évidence soit de la séquence elle-même, comme une sonde, soit de la protéine codée par ladite séquence, tel qu'un ligand comme un anticorps, ou encore de l'activité de cette protéine, comme un marqueur enzymatique, qui peuvent être détectées par tout moyen connu de l'homme du métier, comme un marquage fluorescent ou radioactif.

Les applications du procédé de clonage selon l'invention sont très nombreux et l'on peut citer notamment celles détaillées dans les exemples ci-après :
- Le clonage d'un gène par banque d'expression.
- Le clonage par homologie.
- Le Southern Blot d'identification dénommé par l'Inventeur "identiblot".
- L'étude du polymorphisme.

Aussi, on désignera indifféremment dans ce qui suit par gène ou insert ou séquence, le fragment d'acide nucléique à cloner selon le procédé de l'invention décrit ci-dessus.

Un procédé de clonage d'un gène par banque d'expression selon l'invention comprend les étapes suivantes :
a) On prépare une banque d'ADNc susceptible de contenir ledit gène en insérant ladite banque dans un vecteur substantiellement dépourvu de tout site de clivage par des enzymes de restriction mais conservant :
   - un ou deux sites pour la construction de la banque, et
   - éventuellement au moins deux autres sites, et de préférence seulement deux sites, identiques ou non, et différent du ou des premiers sites et le flanquant, utiles pour sous-cloner le gène une fois celui-ci identifié et cloné.
b) On vérifie la présence du gène recherché dans la banque en transfectant dans une lignée cellulaire qui n'a pas l'activité ou le phénotype recherché et en mesurant sa restauration par une technique permettant de distinguer les cellules transfectées des cellules non transfectées, par exemple par test cytométrique ou enzymatique.
c) On digère la banque indépendamment par au moins 10 et de préférence environ 50 à 70 enzymes de restriction.
d) On transfecte indépendamment les banques mono-digérées de l'étape (c).
e) On teste par tout moyen approprié chacun des lots obtenus à l'étape (d) pour la présence de l'activité associée au gène à cloner et on évalue l'intégrité de la séquence dudit gène afin d'établir la CEM de l'activité associée audit gène.
   On entend par activité associée au gène la détection par tout moyen de la protéine codée par ledit gène ou de l'activité de cette protéine quelle qu'elle soit (ligand, enzyme, induction de tumeur, etc...).
f) On reprend la banque totale de l'étape (a) et on la digère sensiblement simultanément par les environ 50 à 55 enzymes qui, en moyenne, n'affectent pas l'activité mesurée à l'étape (e). En conséquence, statistiquement, tous les fragments constituant la banque seront clivés, à l'exception du fragment recherché.
g) On transforme la banque multi-digérée dans des bactéries compétentes. En conséquence, seuls les vecteurs contenant un fragment non clivé seront transformés dans les bactéries compétentes.
h) On sous-clone en utilisant la ou les enzymes correspondant au(x) site(s) de sous-clonage ménagé(s) dans le vecteur, puis éventuellement on séquence le gène.

Un procédé de clonage par homologie selon l'invention comprend les étapes suivantes :
a) On prépare une banque d'ADNc comme décrit à l'étape (a) précédente.
b) On digère la banque indépendamment par chacune d'au moins 10 et de préférence environ 70 enzymes de restriction.
c) On transforme les produits de la digestion de l'étape (b) dans des bactéries compétentes.
d) On fait pousser les bactéries transformées en milieu sélectif, de façon à produire des banques digérées débarrassées des produits clivés.
e) On clive séparément chacune de ces banques par la ou les enzymes correspondant au(x) site (s) de sous-clonage ménagé(s) dans le vecteur et l'on dépose séparément chacun de ces produits de digestion dans un puits de gel d'agarose ou d'acrylamide.
f) On fait migrer les produits de digestion de l'étape (e), puis l'on transfère sur membrane par exemple de nitro-cellulose, et l'on hybride avec une sonde spécifique du gène à cloner par homologie, ou bien l'on dépose directement les produits de l'étape (d) sur une membrane de nitrocellulose.
g) On analyse la CEM du signal.
e) On fait les multi-digestions correspondantes, de façon à ce que le seul clone résistant soit le vecteur portant le gène à cloner.

Le procédé de clonage par homologie ci-dessus peut être appliqué à l'identification :
a) d'allèles de différentes souches d'animaux de la même espèce, ou de différents individus chez l'homme (souvent très homologues) .
b) d'équivalents géniques présents chez différentes espèces (modérément homologues).
c) d'épissages alternatifs d'un même gène au sein d'un même tissu ou entre différents tissus (homologie totale par sections).
d) de différents membres d'une famille génique, distribuée au sein d'un même tissus ou dans différents tissus. (homologie imprévisible, souvent très forte dans certains domaines).

La version simplifiée de ce procédé consiste à effectuer des dots au lieu de Blots, c'est-à-dire à s'abstenir de cliver les banques mono-digérées par l'enzyme correspondant au site de sous-clonage ménagé dans le vecteur, et de les déposer directement en un point sur une membrane de nitro-cellulose.

Un procédé de Southern Blot d'identification d'inserts selon l'invention permet d'identifier un fragment d'ADN sans avoir à le séquencer même partiellement. Ce procédé comprend les étapes suivantes :
(a) on prépare une banque d'ADN susceptible de contenir ledit insert, consistant à insérer chacun des fragments d'ADN d'un échantillon dans un vecteur substantiellement dépourvu de tout site de clivage par des enzymes de restriction mais conservant :
   - un ou deux sites pour la construction de la banque, et
   - éventuellement au moins deux autres sites, et de préférence seulement deux sites, identiques ou non, et différent du ou des premiers sites et le(s) flanquant, utiles pour sous-cloner l'insert une fois celui-ci identifié et cloné.
b) On digère cette banque par chacune d'au moins 10 et de préférence environ 50 à 70 enzymes de restriction.
c) On transforme les banques monodigérés obtenues à l'étape (b) ci-dessus dans des bactéries compétentes, ou des hôtes équivalents.
d) On fait pousser les bactéries en milieu sélectif, de façon à produire des banques monodigérées débarassées des produits clivés.
e) On clive séparément chacune de ces banques par l'enzyme correspondant aux deux autres sites identiques et différent du premier ou des premiers site(s) et le(s) flanquant, et on dépose les produits de digestion dans les puits d'un gel d'agarose ou d'acrylamide.
f) On fait migrer ce gel et on le transfère sur une membrane par exemple de nitrocellulose.
g) On utilise les inserts à identifier comme sondes marquées, soit un par un, soit plusieurs par plusieurs.
h) On teste par tout moyen approprié chacun des lots obtenus à l'étape (g) pour associer les inserts à identifier à une CEM. Cette CEM correspond à l'action des enzymes de l'étape (b).

Un procédé d'étude du polymorphisme selon l'invention est identique au procédé de Southern Blot d'identification d'inserts mais est caractérisé en ce ce que :
- La banque d'ADN génomique de l'étape (a) est issue du sujet étudié, par exemple d'un malade, ou de sujets de la famille du malade.
- Les inserts utilisés comme sondes sont des marqueurs de polymorphisme déjà décrits.

Le procédé d'étude du polymorphisme selon l'invention trouve son application soit dans le cadre de la recherche de marqueurs du polymorphisme associés à une pathologie, soit dans le cadre d'un diagnostic de cette pathologie.

Une variante du procédé de clonage précédent pour l'étude du polymorphisme d'un individu comprend les étapes suivantes :
a) On définit la CEM de chacun des marqueurs connus de façon à permettre leur identification.
b) On constitue une banque d'ADN génomique du sujet étudié consistant à insérer chacun des fragments d'ADN d'un échantillon dans un vecteur substantiellement dépourvu de tout site de clivage par des enzymes de restriction mais conservant :
   - un ou deux sites pour la construction de la banque, et
   - éventuellement au moins deux autres sites, et de préférence seulement deux sites, identiques ou non, et différent du ou des premiers sites et le(s) flanquant, utiles pour sous-cloner l'insert une fois celui-ci identifié et cloné.
c) On effectue la digestion de la banque par des batteries d'enzymes correspondant aux CEM attribuées aux marqueurs étudiés.
d) On transforme les banques multidigérées dans des bactéries compétentes.
e) On cultive ces bactéries en milieu liquide ou solide contenant l'agent sélectif du plasmide. Si l'allèle recherché éxiste, les bactéries poussent, par contre, si cet allèle n'existe pas, elles ne poussent pas, et l'on dispose ainsi d'un profil pour chaque allèle.

Avantageusement, à l'étape (b), on prépare une banque dont les fragments ont une longueur moyenne de 1000 à 4000 et de préférence de 2000 nucléotides.

Un mode de réalisation de la variante ci-dessus permet également la détection des nombreux allèles de segments polymorphes, comme par exemple la gp120 des virus HIV ou la p53 d'oncogènes cellulaires, dans le cadre de travaux de recherche ou d'un diagnostic. Le segment concerné est avantageusement amplifié par PCR et cloné dans le plasmide, puis le proçédé est identique à celui décrit précédemment et exposé en détail dans l'exemple 6 ci-après.

D'autres avantages et caractéristiques du procédé de l'invention apparaitront dans la description qui suit se rapportant à des exemples détaillés de mise en oeuvre du procédé de l'invention dans diverses applications qui ne sauraient être considérés comme une limitation quelconque de l'invention.

### Exemple 1 La préparation et l'exploitation d'une banque d'expression.

Une première application du procédé de l'invention se rapporte à la préparation et à l'exploitation d'une banque d'expression.

Cette technique suscite depuis le milieu des années 1980 un grand intérêt. De nombreux gènes, comme ceux codant pour des récepteurs aux cytokines, des marqueurs de surface des lymphocytes, des protéines liant l'ADN, etc... ont été identifiés par cette technique (U. Gubler et al., Annals of the N. Y. Academy of Science 795 : 36-40, 1996 ; D. Pennica et al. PNAS 92(4) : 1142, 1995 ; R. M. O'Brien et al., Biochemical Journal 312 : 17 - 21, 1995). Toutefois, les techniques de clonage par banque d'expression sont toujours fastidieuses et difficilement reproductibles à ce jour.

Les applications actuelles des banques d'expression concernent l'identification du gène codant pour une protéine, dont les moyens de mise en évidence peuvent être regroupé en quatre catégories principales :
- les anticorps,
- les liaisons protéine/protéine autre qu'anticorps/antigène,
- les oligonucléotides marqués par exemple par fluorescence dans le cas où la protéine recherchée est une protéine se liant à l'ADN,
- les tests d'activité de la protéine, quelle qu'elle soit.

La banque contenant le gène codant pour la protéine recherchée peut être transformée dans des bactéries ou des levures, et l'on utilise l'anticorps, la protéine ou l'oligonuctéotide marqué pour balayer la banque à la recherche de la colonie qui l'exprime.. Ces systèmes marchent souvent très mal, car les protéines n'ont pas la même conformation et les mêmes modifications post-traductionnelles chez les bactéries ou levures et chez les cellules mammifères.

Le problème de la transfection de banques dans les cellules mammifères réside dans le fait que, à la différence des systèmes de bactéries ou de levures, plusieurs plasmides sont intégrés dans chaque cellule. Pour contenir cette difficulté, il est nécessaire d'utiliser des techniques de fractionnement successif des banques, ou de tris cytofluorimétriques répétés (T. Kitamura et al., PNAS 92 (20) : 9146, 1995 ; D. R. Gehlert et al., Molecular Pharmacology, 49 (2) : 224, 1996). Chacune de ces deux techniques représente un travail important, très long (plusieurs semaines), qui n'est pas toujours conclusif, et qui n'est pas adapté au clonage simultané deux inserts ou plus.

Le procédé de l'invention est beaucoup plus simple et moins coûteux que tous ceux de l'art antérieur cité ci-dessus. De plus, il peut être appliqué au clonage simultané de plusieurs inserts. Ce dernier point revêt une importance majeure lorsqu'on sait que beaucoup de protéines de surface sont composées de plusieurs chaînes et n'atteignent la surface que lorsque toutes les chaînes sont produites, par exemple dans le cas des complexes majeurs d'histocompatibilité. Or aucune des techniques de l'art antérieur basée sur les banques d'expression ne permet d'avoir accès à ces protéines.

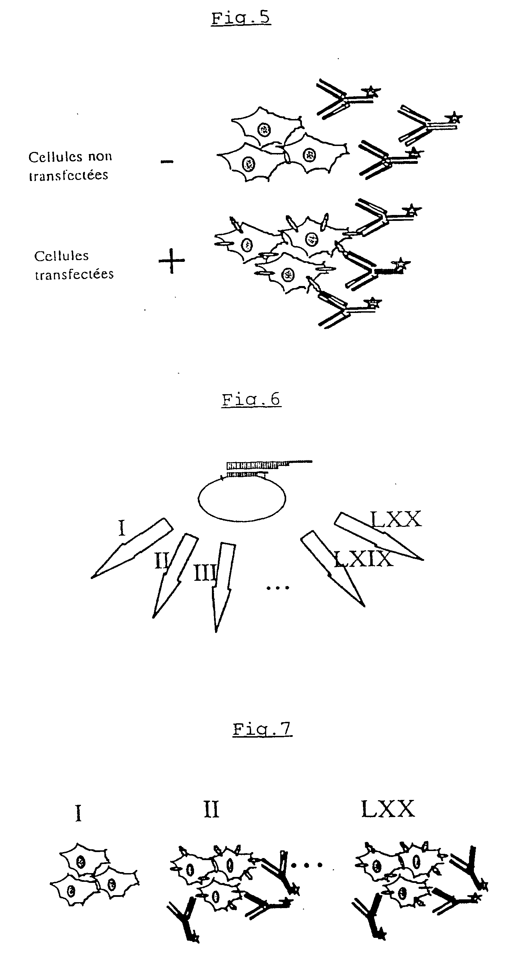

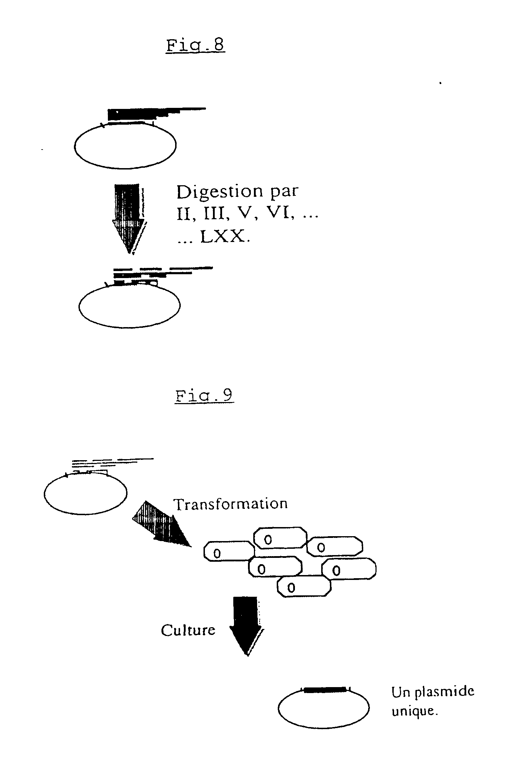

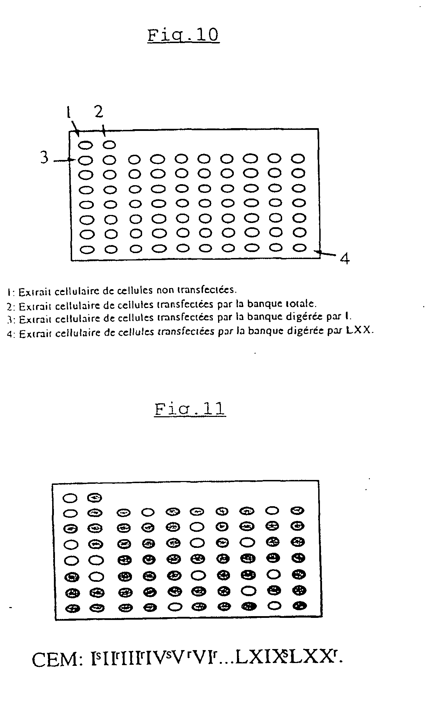

Un procédé de l'invention pour la constitution de banques d'expression comprend les étapes suivantes :
a) On prépare une banque en insérant dans le site A du vecteur l'ADN complémentaire du tissu ou de la lignée cellulaire d'intérêt. La figure 2 en annexe est une représentation schématique de cette étape.
b) On vérifie la présence du gène recherché dans la banque en transfectant dans une lignée cellulaire qui n'a pas l'activité ou le phénotype recherché et en mesurant sa restauration, par exemple avec un anticorps, de façon à distinguer les cellules transfectées (+) des cellules non transfectées (-). La figure 4 et 5 en annexe sont une représentation schématique de cette étape.
   Avantageusement, on utilise des cellules COS, classiquement mise en oeuvre pour les transfections.
c) On digère la banque indépendamment par chacune des 50 à 70 enzymes de restriction disponibles. On obtient ainsi 50 à 70 tubes. La figure 6 en annexe est une représentation schématique de cette étape.
d) On transfecte indépendamment les 50 à 70 banques mono-digérées, et tester la présence de l'activité recherchée dans chacun de ces 50 à 70 lots de cellules transfectées. La figure 7 en annexe est une représentation schématique de cette étape.
e) On établit ainsi la CEM de l'activité recherchée. Si la digestion par l'enzyme I n'altère pas l'activité de l'insert présent dans la banque, on notera I^{r}. Si elle l'altère, on notera I^{s}.
   On obtient, dans l'exemple de la figure 7 en annexe, la CEM suivante: I^{s} II^{r}...LXX^{r}.
   En moyenne, on estime que 55±4 enzymes sur 70 auront un r, et 15±4 auront un s pour un insert de 1 kb. En effet, la probabilité de clivage par une enzyme de restriction à site hexanucluéotidique, à une position donnée prise au hasard, est p = 1/4⁶ = 1/4096. Sur un gène dont la taille est n nucléotides, la probabilité théorique d'avoir 1, 2, 3, ... clivages suit une loi binomiale de probabilité p et de nombre d'évènements n. La probabilité de n'avoir aucun clivage est Cₙ⁰pⁿ(1-p)⁰, Dans le cas où n = 1000, on obtient une probabilité de 78,3%. La probabilité d'avoir un clivage ou plus est d'environ 21,7 %. Le nombre moyen d'enzymes ne clivant pas est donc de 0,783 x 70 = 55, et l'écart type est proche de 4.
f) On reprend la banque totale et on la digère sensiblement simultanément par les 55 enzymes qui n'affectent pas l'activité mesurée qui est associée à "r" pour l'insert recherché. La figure 8 en annexe est une représentation schématique de cette étape.
   En pratique, cette digestion ne peut être totalement simultanée pour des raisons de compatibilité de tampons, et l'on est obligé de 2 ou 3 multi-digestions successives correspondant aux deux ou trois tampons choisis.
   La probabilité pour un insert pris au hasard d'être clivé par x de ces 55 enzymes suit aussi une loi binomiale, de probabilité 0,783 et de nombre d'événements 55. La probabilité pour un insert de n'être clivé par aucune des 55 enzymes est donc C₅₅⁰ x (0,783)⁵⁵ x (1 - 0,783)⁰ = (0,783)⁵⁵ = 1,4.10⁻⁶.
   Il ne reste donc, en moyenne, que l'insert recherché, plus 1,4.10⁻⁶ x 10⁵ = 0,14 insert parasite.
   L'utilisation partielle de la CEM, correspondant uniquement aux enzymes associées à un r, est suffisante pour isoler le gène recherché, lequel est déjà pur à plus de 85%.
g) On transforme la banque multi-digérée dans des bactéries compétentes. Avantageusement on étale sur petri et l'on vérifie par miniprep que les inserts sont bien sensibles aux enzymes notées "s" lors de l'établissement de la CEM. La figure 9 en annexe est une représentation schématique de cette étape.
h) On sous-clone en utilisant l'enzyme B dans un vecteur d'étude, tel que Bluescript. Puis avantageusement on séquence.

Le modèle décrit à l'étape (e) ci-dessus est conforme à la réalité, mais ne représente qu'une moyenne. En effet, la probabilité de clivage varie d'une enzyme de type site hexanucléotidique à une autre.

Chacune de ces 8 étapes ne demande que très peu de temps, puisque le procédé ci-dessus devrait peut être réalisé en 16 jours dont 10 de travail.

Le procédé de l'invention permet notamment de générer des banques d'expression pour de nombreux tissus et lignées, puis en faire les 50 à 70 simples digestions, les transfecter, faire l'extrait cellulaire de ces cellules transfectées, et les déposer sur une membrane comme représenté à la figure 10.

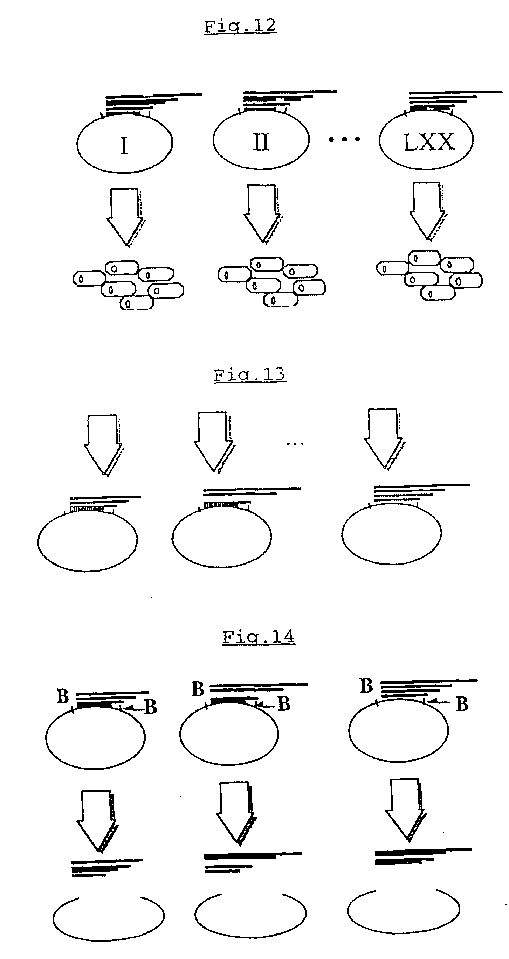

Il ne reste alors plus qu'à révéler la membrane par exemple avec un anticorps selon la technique dite de Western blot, afin d'obtenir immédiatement la CEM de l'insert recherché comme représenté sur la figure 11. La banque est alors multi-digérée en fonction de la CEM obtenue, puis on transforme des bactéries compétentes avec ce produit multi-digéré, et le gène est cloné en 3 jours au lieu de plusieurs mois par les techniques de l'art antérieur.

On peut conserver ces membranes ad vitam aeternam à 4°C ou congelées.

L'exemple ci-dessus est basé sur l'utilisation d'un anticorps spécifique mais d'autres systèmes de révélation du phénotype associé à la CEM peuvent être utilisés, comme un test enzymatique.

### Exemple 2 : Le clonage par homologie.

Le clonage par homologie est intensivement utilisé par de très nombreux laboratoires de biologie moléculaire pour l'identification :
a) d'allèles de différentes souches d'animaux de la même espèce, ou de différents individus chez l'homme (souvent très homologues).
b) d'équivalents géniques présents chez différentes espèces (modérément homologues).
c) d'épissages alternatifs d'un même gène au sein d'un même tissu ou entre différents tissus (homologie totale par sections).
d) de différents membres d'une famille génique, distribuée au sein d'un même tissus ou dans différents tissus. (homologie imprévisible, souvent très forte dans certains domaines).

Les stratégies utilisées dans l'art antérieur sont essentiellement les deux suivantes (M. Parmentier et al., Nature, 355 : 453, 1992) :
- La PCR par homologie et ses dérivés, lesquels soulèvent le problème du choix des amorces alors que l'on ne connaît pas les parties conservées. La fenêtre entre le bruit de fond aspécifique et les vraies amplifications homologues est étroite. De plus, la partie amplifiée ne représente le plus souvent pas tout le gène et on est alors obligé d'aller chercher les morceaux manquants, et notamment la partie en 5', par des techniques laborieuses comme la PCR ancrée.
- L'hybridation de banques existant actuellement. Cette méthode est efficace, mais nécessite beaucoup de travail. En outre, elle ne s'applique qu'à l'identification d'allèles (a) ou d'équivalents géniques (b). Pour l'identification des épissages alternatifs (c)
ou des différents membres d'une famille génique (d), on reclone toujours l'ADNc majoritaire, donc dans la plupart des cas, le gène dont on dispose déjà.

Le procédé de l'invention permet une fois qu'on a cloné un gène, de produire une sonde qui s'hybride sur des gènes homologues. On utilise donc cette sonde pour pêcher ses homologues par hybridation à faible stringence.

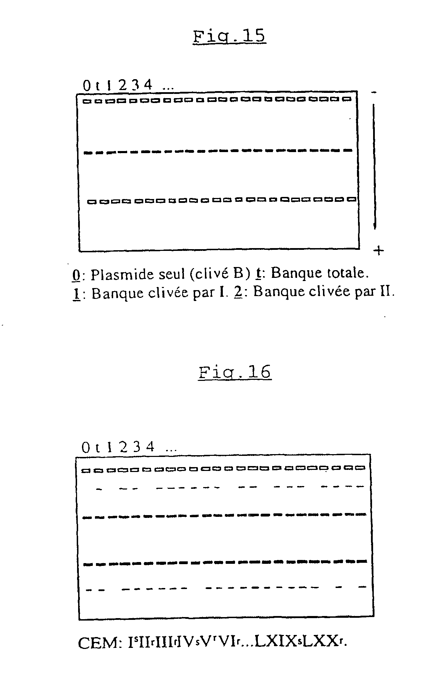

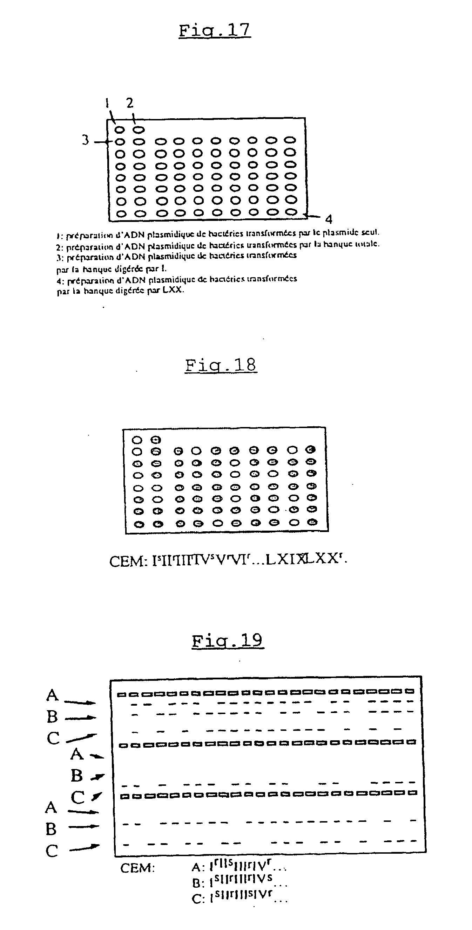

Le procédé de clonage par homologie selon l'invention comprend les étapes suivantes .
a) On prépare une banque d'ADNc comme décrit à l'étape (a) de l'exemple 1.
b) On digère la banque indépendamment par chacune des 70 enzymes de restriction comme comme décrit à l'étape (c) de l'exemple 1.
c) On transforme ces 50 à 70 digestions dans des bactéries compétentes. La figure 12 en annexe est une représentation schématique de cette étape.
d) On fait pousser les bactéries transformées en milieu sélectif, de façon à produire des grandes quantités des banques digérées débarrassées des produits clivés (non transformants en raison de leur linéarisation). Les éléments clivés sont désormais absents des banques. La figure 13 en annexe est une représentation schématique de cette étape.
e) On clive séparément chacune de ces banques par l'enzyme B. La figure 14 en annexe est une représentation schématique de cette étape.
   On dépose alors séparément chacun de ces 50 à 70 produits de digestion dans un puits de gel d'agarose, comme représenté à la figure 15 en annexe.
   Puis, on fait migrer, l'on transfère sur nitro-cellulose, et l'on hybride avec la sonde, comme représenté à la figure 16 en annexe.
f) On analyse la CEM du signal. Ainsi, si une bande est présente dans la piste "banque non digérée", mais absente dans la piste "banque digérée par I", c'est que l'insert hybridé est sensible à l'enzyme I, et ainsi de suite.
g) On fait les multi-digestions correspondantes, et le seul plasmide résistant est le plasmide recherché. Avantageusement, on peut ensuite faire des minipreps avec ce plasmide en utilisant les enzymes "s" pour confirmer.

Ce procédé peut être utilisé industriellement, en proposant les Blots correspondant à de nombreuses banques, comme celles réalisées dans l'exemple 1. Ainsi, en disposant de la sonde et des Blots judicieusement choisis, il devient quasi immédiat de trouver tous les allèles, correspondants d'espèce, épissages alternatifs et isotypes (deux jours de travail, sans compter le séquençage).

Une version simplifiée de ce procédé consiste à effectuer des Dots au lieu de Blots, c'est-à-dire à s'abstenir de cliver les banques mono-digérées par l'enzyme B, et à les déposer directement en un point sur une membrane de nitro-cellulose. Lors de l'hybridation avec la sonde, les points générant un signal, par exemple radioactif, correspondraient aux enzymes pour lesquelles le plasmide serait résistant. Ceux ne retenant plus de signal correspondraient aux enzymes pour lesquelles l'insert est sensible. L'analyse est alors proche de celle développée dans l'exemple 1, comme représentée aux figures 17 et 18 en annexe.

Cette version simplifiée est cependant insuffisante pour cloner des isotypes ou des épissages alternatifs exprimés dans une même cellule.

### Exemple 3 : Southern Blot d'identification "identiblot".

Les applications précédentes du procédé de l'invention visent à simplifier et rendre plus rapide le clonage par expression ou par homologie et en offrant de nouvelles possibilités.

Le Southern Blot d'identification propose un raccourci qui faciliterait les travaux du chercheur impliqué dans les techniques de clonage moléculaire.

En effet, Le cas est fréquent dans l'art antérieur où une stratégie de clonage aboutit à l'obtention de nombreux inserts, parmi lesquels se trouve le gène recherché et de nombreux parasites. Or dans l'art antérieur, pour identifier un insert parmi tous ces parasites, il est est indispensable de le séquencer, au moins partiellement, ce qui représente un travail considérable. Le séquençage est une technique fastidieuse et trop puissante pour ce simple usage d'identification d'inserts, en effet la lecture de 10 nucléotides suffit le plus souvent à identifier un insert. Un technique moins puissante, mais moins fastidieuse, utilisant la DMD, pourrait lui être substituée.

Ainsi, comme décrit précédemment, il est possible de faire des Southern Blots de banques d'ADN génômique de façon industrielle. Une représentation schématique de la préparation d'une telle banque d'ADN génomique est donnée à la figure 3 en annexe. Ce Southern Blot, rebaptisé "identiblot" dans le cadre de la présente invention est suffisamment informatif pour permettre l'identification du fragment d'ADN homologue de la sonde.

Ce southern blot est même cent millions de fois trop informatif. En effet, la banque génomique est constituée de 4 millions d'inserts différents de 1 kb de long. Pour les mêmes raison que celles exposées précédemment, un insert pris au hasard sera résistant à 55 enzymes sur 70 et sensible à 15. Le nombre de combinaison enzymatique possible est donc C₇₀¹⁵, soit 70!/(55!15!) = 7,2.10¹⁴. Ce nombre est plus de cent millions de fois supérieur à la taille de la banque. Le fait de considérer que les inserts ont tous une taille différente ajoute encore des possibilités. Chaque insert de la banque est donc associé à une CEM originale.

Ce procédé présente un intérêt majeur dans le cas de stratégies de clonages aboutissant à de forts taux de clones faux positifs, par exemple dans les cas de l'utilisation de banques soustractives ou de stratégies de clonage par insertion.

En effet, à partir des cinquante clones étudiés, il suffit de préparer une "sonde multiplex", c'est à dire un marquage commun de cinquante inserts (dans un seul tube), et une seule hybridation du filtre de nitro-cellulose. On obtient l'identité des cinquante inserts d'un seul coup, en comparant leurs CEM avec celles préalablement entrées dans une base de données informatique.

La figure 19 en annexe représente un exemple où trois sondes A, B, et C ont été utilisées simultanément. Les inserts génomiques correspondant à des CEM non décrites, et eux seuls, seront clonées par exemple par multi-digestion, puis séquencés.

A l'inverse du cas des banques d'ADNc, dont la variété est insondable, il est suffisant de produire un seul type de Blot, pour chacune des 10 espèces étudiées couramment en biologie : humain, souris, rat, drosophile, tabac, levure, etc...

### Exemple 4 : Etude du polymorphisme humain.

Les travaux de recherche menés dans le cadre de l'étude du polymorphisme nécessitent l'utilisation d'un nombre croissant de marqueurs génétiques. Leur utilisation dans le cadre du diagnostic des maladies génétiques est également en forte augmentation. Il est probable que dans un futur proche des cartes personnelles de prédisposition génétique pourront être établies, de façon à ce que chacun évite de s'exposer à certains risques environnementaux, comme le tabac en cas de prédisposition au cancer du poumon, le sucre pour les terrains diabétiques, etc....

Les techniques disponibles dans l'art antérieur permettant d'étudier le polymorphisme sur un allèle sont principalement la PCR, le Southern Blot et l'étude des marqueurs de l'ADN satellite (potentiellement utilisés en combinaison) . La méthode de l'invention constitue donc une alternative, plus efficaces, à ces techniques.

La mise en oeuvre du procédé de clonage selon l'invention pour l'étude du polymorphisme humain est proche de celle de l'exemple 3 ci-dessus. La différence majeure réside dans le fait qu'il est nécessaire de réaliser la banque, ses digestions, la migration sur gel et le transfert sur membrane, pour chaque sujet.

Ce procédé permet également de localiser très rapidement l'origine d'une maladie génétique par l'analyse des différents membres de la famille hébergeant la pathologie. Dans une famille dite "à risque" pour une pathologie, il convient de faire les Identiblots correspondant aux différents membres de la famille, et de les tester au moyen de polysondes faites à partir des marqueurs génétiques déjà existants. En un jour, on peut hybrider au moins vingt membranes, soit obtenir l'allèle de 1000 marqueurs. Les facteurs génétiques multiples pourraient être définis avec cette technique.

Cette application du procédé de l'invention implique un investissement en temps de l'ordre de plusieurs heures de travail, mais est rapidement rentabilisé par la possibilité de tester les marqueurs de prédisposition génétique 50 par 50, au moyen de sondes multiplex préparées en routine et constituées du nombre équivalent de marqueurs.

### Exemple 5 : Deuxième approche de l'étude du polymorphisme humain selon l'invention.

Cette seconde mise en oeuvre du procédé de l'invention à l'étude du polymorphisme concerne l'application de la DMD à l'étude des marqueurs génétiques par RFLP. Le procédé selon l'invention comprend alors les étapes suivantes :
a) On a préalablement définis la CEM de chacun des marqueurs connus de façon à permettre leur identification. Cette identification est faite une fois pour toutes et un polymorphisme est donc caractérisé par une variation de la CEM.
b) On constitue une banque d'ADN génomique du sujet étudié dans le vecteur précédemment décrit. Comme indiqué précédemment, il peut être avantageux que les vecteurs seuls ligués sur eux-mêmes soient éliminés. Avantageusement, on prépare une banque dont les fragments ont une longueur moyenne de 2000 nucléotides.
c) On effectue la digestion de la banque par des batteries d'enzymes correspondant aux CEM attribuées aux marqueurs étudiés.
   Ainsi, par exemple, dans le premier puits, on met les cinquante enzymes qui vont digérer toute la banque sauf un premier marqueur. Dans le second puits, on met une autre batterie d'enzymes pour un second marqueur, etc...
d) On transforme les banques multidigérées dans des bactéries compétentes.
e) On cultive en milieu solide (boite de petri) contenant l'agent sélectif du plasmide de façon à ce que si l'allèle recherché existe, les bactéries poussent. Par contre, si cet allèle n'existe pas, elles ne poussent pas. On dispose ainsi d'un profil pour chaque allèle, et l'on peut étudier un nombre illimité de marqueurs en une seule fois.

Ce procédé peut être automatisé en préparant des plaques de 96 puits contenant tous les mélanges enzymatiques, ces plaques sont stockées en congélateur. Différents types de plaques pourraient être produites:
- Marqueurs répartis sur tout le génome.
- Marqueurs répartis sur un seul chromosome.
- Marqueurs répartis sur une région précise.
- Marqueurs reliés aux risques de pathologies.
- Etc...

On distribue dans chaque puits une quantité fixe d'ADN sous forme de banque, puis l'on incube à 37°C pour les digestions. On rajoute alors les bactéries compétentes, et on suit le mécanisme classique de transformation (choc thermique, incubation sans agent de sélection, etc...). On prélève à la pipette 96 canaux et l'on dépose sur un pétri. La lecture est effectuée à l'oeil ou à l'aide d'un spectrophotomètre.

Cette seconde approche offre l'avantage d'être facilement automatisable, et confère à grande échelle un gain de temps très important. En effet, il faut tester un grand nombre de marqueurs pour chaque individu, car la création de la banque représente un investissement en travail. En outre, elle permet de s'exonérer de toute radioactivité, coûteuse et nocive.

### Exemple 6 : Troisième approche de l'étude du polymorphisme humain selon l'invention.

Cette variante du procédé d'étude du polymorphisme humain selon l'invention se rapporte à l'application de la DMD à l'étude des différents allèles d'un seul marqueur.

Dans l'art antérieur, ces différentiations se font essentiellement par séquençage. Dans un futur proche, les puces d'ADN (T. Pastinen et al., Genome Research 7 : 606-14, 1997 ; J. G. Hacia et al., Nature Genetics, 14 : 441, 1996), permettront d'automatiser ces travaux. Ceux-ci sont particulièrement utiles dans le cas de l'examen de la gp120 du virus HIV ou d'oncogènes cellulaires tels p53, présents sous de nombreux allèles.

Conformément à la présente invention, cette variante consiste à préalablement définir la CEM de chacun des allèles du segment étudié. Cette identification est réalisée une fois pour toute et se limite aux allèles comportant au moins un site de restriction de différence. Puis on amplifie par PCR le fragment étudié. On clone ledit fragment dans le vecteur de l'invention. Ce procédé est donc très proche de celui de l'exemple 5, mais avec une banque comportant seulement un ou deux inserts correspondant aux deux copies du gène présent chez un individu. La suite de ce procédé est identique à celle de l'exemple 5.

Ce travail peut être réalisé simultanément sur plusieurs inserts à la fois.

## Revendications

1. Procédé, de clonage d'un fragment d'acide nucléique, **caractérisé en ce qu'**il comprend les étapes suivantes :
a) On prépare une banque d'ADN susceptible de contenir ledit fragment d'acide nucléique à cloner, en insérant des fragments d'ADN d'un échantillon dans un vecteur dépourvu de tout site de clivage par des enzymes de restriction dont le site comprend de 5 à 6 nucléotides correspondantes à celles utilisées pour les digestions de la banque, mais conservant au moins un ou deux sites pour la construction de la banque.
b) On réalise la digestion en parallèle de la banque par au moins 10 enzymes de restriction dont le site comprend de 5 à 6 nucléotides, de façon obtenir autant de banques mono-digérées que d'enzymes utilisées.
c) On teste chacune des banques monodigérées obtenues à l'étape précédente pour évaluer l'intégrité du fragment d'acide nucléique à cloner et ainsi établir sa caractéristique enzymatique multiple (CEM).
d) On reprend la banque totale initiale et on la digère simultanément par les enzymes de restriction qui n'affectent pas l'intégrité du fragment d'acide nucléique à cloner, de façon obtenir une banque multi-digérée.
e) On isole le clone résistant contenant le fragment d'acide nucléique à cloner.

2. Procédé de clonage d'un fragment d'acide nucléique selon la revendication 1, **caractérisé en ce qu'**à l'étape (b) on digère la banque par 50 à 70 enzymes de restriction.

3. Procédé de clonage d'un fragment d'acide nucléique selon l'une des revendications 1 ou 2, **caractérisé en ce que** le vecteur de clonage de l'étape (a) comprend en outre au moins deux autres sites, identiques ou non, et différent du ou des premiers sites et le(s) flanquant, utiles pour sous-cloner le fragment d'acide nucléique une fois celui-ci isolé.

4. Procédé de clonage d'un fragment d'acide nucléique selon l'une des revendications 1 à 3, **caractérisé en ce qu'**il comprend entre les étapes (b) et (c), l'étape suivante :
b') On transfecte indépendamment les banques mono-digérées dans des hôtes cellulaires de façon à obtenir des lots correspondants d'hôtes cellulaires.

5. Procédé selon l'une quelconque des revendications précédentes, **caractérisé en ce qu'**il comprend entre les étapes (a) et (b), l'étape suivante :
a') On vérifie la présence du fragment d'acide nucléique à cloner dans la banque en transfectant dans un hôte cellulaire qui n'a pas ledit fragment et en testant la présence du fragment dans ledit hôte.

6. Procédé selon l'une quelconque des revendications précédentes, **caractérisé en ce qu'**il comprend entre les étape (d) et (e) l'étape suivante :
d') On transforme la banque multi-digérée de l'étape (d) dans des hôtes compétents, de façon à vérifier la nature des fragments clonés.

7. Procédé selon l'une quelconque des revendications 1 à 6, **caractérisé en ce** la banque d'ADN contient de 1 à 10⁸ fragments différents de l'ordre de 0,1 kb à 5 kb chacun.

8. Procédé selon l'une quelconque des revendications précédentes, **caractérisé en ce que** les tests réalisés aux étapes (c) et/ou (a') pour vérifier l'intégrité de la séquence d'acide nucléique à cloner sont tout moyen de mise en évidence soit de la séquence elle-même, comme une sonde, soit de la protéine codée par ladite séquence, tel qu'un ligand comme un anticorps, ou encore de l'activité de cette protéine, comme un marqueur enzymatique qui peuvent être détectées par tout moyen connu de l'homme du métier, comme un marquage fluorescent ou radioactif.

9. Procédé de clonage d'un gène par banque d'expression selon l'une quelconque des revendications 1 à 8, **caractérisé en ce qu'**il comprend :
- l'étape (a),
- l'étape (a'), consistant à vérifier la présence du gène recherché dans la banque en transfectant dans une lignée cellulaire qui n'a pas l'activité ou le phénotype recherché et en mesurant sa restauration de façon à, distinguer les cellules transfectées des cellules non transfectées,
- l'étape (b),
- l'étape (b'), consistant à transfecter indépendamment les banques mono-digérées de l'étape (b),
- étape (c), consistant à tester chacun des lots obtenus à l'étape (b') pour la présence de l'activité associée au gène à cloner et on évalue l'intégrité de la séquence dudit gène afin d'établir la CEM de l'activité associée audit gène,
- l'étape (d),
- l'étape (d'), consistant à transformer la banque multi-digérée dans des bactéries compétentes,
- l'étape (e).

10. Procédé de clonage par homologie selon l'une quelconque des revendications 1 à 8, **caractérisé en ce qu'**il comprend :
- l'étape (a),
- l'étape (b),
- l'étape (b'), consistant à transformer les produits de la digestion de l'étape (b) dans des bactéries compétentes.
- l'étape (c), consistant à faire pousser les bactéries transformées en milieu sélectif de façon à produire des grandes quantités des banques digérées débarrassées des produits clivés, puis à cliver séparément chacune de ces banques par l'enzyme correspondant au site de sous-clonage présent dans le vecteur, à déposer séparément chacun des produits de digestion dans un puits de gel d'agarose, à faire migrer les produits de digestion, à les transfèrer sur membrane et à les hybrider avec une sonde spécifique du gène à cloner par homologie afin d' établir sa CEM,
- l'étape (d),
- l'étape (e).

11. Application du procédé selon la revendication 9 à l'identification :
- d'allèles de différentes souches d'animaux de la même espèce, ou de différents individus chez l'homme,
- d'équivalents géniques présents chez différentes espèces,
- des épissages alternatifs d'un gène.
- de différents membres d'une famille génique.

12. Procédé de Southern Blot d'identification d'inserts mettant en oeuvre un procédé de clonage d'une séquence d'acide nucléique selon l'une quelconque des revendications 1 à 9, **caractérisé en ce qu'**il comprend :
- l'étape (a),
- l'étape (b),
- l'étape (b'), consistant à transformer les banques monodigérés obtenues à l'étape (b) ci-dessus dans des bactéries compétentes, ou des hôtes équivalents,
- l'étape (c), consistant à faire pousser les bactéries en milieu sélectif, de façon à produire des banques digérées débarassées des produits clivés, puis à cliver séparément chacune de ces banques par la ou les enzymes correspondant: au (x) site(s) de sous-clonage flanquant le ou les sites de construction de la banque, et à déposer les produits de digestion dans les puits d'un gel d'agarose ou d'acrylamide, à faire migrer ce gel et à le transférer sur une membrane, et à les hybrider avec les insères à identifier utilisés comme sonde marquée, soit un par un, soit plusieurs par plusieurs afin d'établir leur CEM,

13. Procédé d'étude du polymorphisme mettant en oeuvre un procédé de Southern Blot d'identification d'inserts selon la revendication 12, **caractérisé en ce que** :
- La banque d'ADN génomique de l'étape (a) est issue du sujet étudié.
- Les inserts utilisés comme sondes à l'étape (c) sont des marqueurs de polymorphisme.

14. Procédé d'étude du polymorphisme **caractérisé en ce qu'**il comprend :
- les étapes (a) à (c) du procédé selon l'une des revendications 1 à 10, consistant à définir la CEM de chacun des marqueurs connus, puis la constitution d'une banque d'ADN génomique,
- l'étape (d), consistant à effectuer la digestion par des batteries d'enzymes correspondant aux CEM attribuées aux marqueurs étudiés,
- l'étape (d'), consistant à transformer les banques multi-digérées dans des bactéries compétentes,
- l'étape (e), consistant à cultiver ces bactéries en milieu liquide ou solide contenant un agent sélectif du plasmide.

## Patentansprüche

1. Verfahren zum Klonen eines Nucleinsäurefragments, **dadurch gekennzeichnet, dass** das Verfahren die folgenden Schritte umfasst:
a) Herstellen einer DNA-Bank, die geeignet ist, um das zu klonierende Nucleinsäurefragment zu enthalten, indem DNA-Fragmente einer Probe in einen Vektor eingefügt werden, von dem sämtliche Spaltungsstellen durch Restriktionsenzyme entfernt wurde, bei denen die Stelle 5 bis 6 Nucleotide enthält, die jenen entsprechen, die für die Digestionen der Bank verwendet werden, wobei jedoch wenigstens eine oder zwei Stellen für den Aufbau der Bank beibehalten werden.
b) Durchführen der Digestion parallel zu der Bank mittels wenigstens 10 Restriktionsenzymen, bei denen die Stelle 5 bis 6 Nucleotide enthält, so dass die Anzahl der gewonnenen monoaufgeschlossenen Banken gleich der Anzahl der verwendeten Enzyme ist.
c) Testen jeder der in dem vorhergehenden Schritt gewonnenen monoaufgeschlossenen Banken, um die Integrität des zu klonierenden Nucleinsäurefragments zu bewerten und dementsprechend dessen multiples enzymatisches Merkmal (CEM = Caractéristique enzymatique multiple) zu bestimmen.
d) erneute Verwendung der anfänglichen Gesamtbank und gleichzeitiges Aufschließen derselben durch Restriktionsenzyme, die die Integrität des zu klonierenden Nucleinsäurefragments nicht beeinträchtigen, so dass eine mehrfach aufgeschlossene Bank gewonnen wird.
e) Isolieren des resistenten Klons, der das zu klonierende Nucleinsäurefragment enthält.

2. Verfahren zur Klonierung eines Nucleinsäurefragments nach Anspruch 1, **dadurch gekennzeichnet, dass** in dem Schritt (b) die Bank durch 50 bis 70 Restriktionsenzyme aufgeschlossen wird.

3. Verfahren zur Klonierung eines Nucleinsäurefragments gemäß einem der Ansprüche 1 oder 2, **dadurch gekennzeichnet, dass** der Klonierungsvektor des Schritts (a) ferner wenigstens zwei weitere Stellen aufweist, die miteinander identisch sind oder auch nicht, sich von der oder den ersten Stellen und de(n) flankierenden unterscheiden und sich für eine Subklonierung des Nucleinsäurefragments eignen, sobald dieses isoliert ist.

4. Verfahren zur Klonierung eines Nucleinsäurefragments gemäß einem der Ansprüche 1 bis 3, **dadurch gekennzeichnet, dass** zwischen den Schritten (b) und (c) der folgende Schritt eingefügt ist:
b') unabhängiges Transfizieren der monoaufgeschlossenen Banken in zelluläre Wirte, um Partien? zu erhalten, die den zellulären Wirten entsprechen.

5. Verfahren nach einem der vorhergehenden Ansprüche, **dadurch gekennzeichnet, dass** zwischen den Schritten (a) und (b) der folgende Schritt eingefügt ist:
a') Verifizieren der Anwesenheit des zu klonierenden Nucleinsäurefragments in der Bank durch Transfizieren in einen zellulären Wirt, der das Fragment nicht aufweist, und Testen der Anwesenheit des Fragments in dem Wirt.

6. Verfahren nach einem der vorhergehenden Ansprüche, **dadurch gekennzeichnet, dass** dieses zwischen dem Schritt (d) und (e) den folgenden Schritt enthält:
d') Transformieren der mehrfach aufgeschlossenen Bank des Schritts (d) in kompetente Wirte, um die Natur der klonierten Fragmente zu verifizieren.

7. Verfahren nach einem der Ansprüche 1 bis 6, **dadurch gekennzeichnet, dass** die DNA-Bank 1 bis 10³ unterschiedliche Fragmente der Größenordnung 0,1 kb bis 5 kb enthält.

8. Verfahren nach einem der vorhergehenden Ansprüche, **dadurch gekennzeichnet, dass** die in den Schritten (c) und/oder (a') durchgeführten Tests zum Verifizieren der Integrität der zu klonierenden Nucleinsäuresequenz allesamt Mittel zum Nachweis sind, sei es für die Sequenz selbst, als Sonde, sei es für das durch die Sequenz kodierte Protein, von der Art eines Liganden in Form eines Antikörpers, oder auch die Aktivität diese Protein als enzymatischer Marker, die durch beliebige dem Fachmann bekannte Mittel als fluoreszierende oder radioaktive Markierung erfasst werden können.

9. Verfahren zur Klonierung eines Gens durch Expressionsbanken nach einem der Ansprüche 1 bis 8, **dadurch gekennzeichnet, dass** dieses umfasst:
- den Schritt (a),
- den Schritt (a'), zu dem es gehört, die Anwesenheit des gewünschten Gens in der Bank zu verifizieren, indem in eine Zelllinie transfiziert wird, die nicht die Aktivität oder den gewünschten Phänotypus aufweist, und indem deren Wiederherstellung gemessen wird, um die transfizierten Zellen von den nicht transfizierten Zellen zu unterscheiden,
- den Schritt (b),
- den Schritt (b'), zu dem es gehört, die monoaufgeschlossenen Banken des Schritts (b) unabhängig zu transfizieren,
- den Schritt (c), zu dem es gehört, jede der in Schritt (b') gewonnenen Partien auf die Anwesenheit der Aktivität zu testen, die dem zu klonierenden Gen zugeordnet ist, und die Integrität der Sequenz des Gens zu ermitteln, um die CEM der dem Gen zugeordneten Aktivität zu bestimmen,
- den Schritt (d),
- den Schritt (d'), zu dem es gehört, die mehrfach aufgeschlossene Bank in kompetente Bakterien umzuwandeln,
- den Schritt (e).

10. Verfahren zur Klonierung durch Homologie nach einem der Ansprüche 1 bis 8, **dadurch gekennzeichnet, dass** dieses umfasst:
- den Schritt (a),
- den Schritt (b),
- den Schritt (b'), zu dem es gehört, die Digestionsprodukte des Schritts (b) in kompetente Bakterien umzuwandeln,
- den Schritt (c), zu dem es gehört, die transformierten Bakterien mit einem selektiven Milieu zusammen zu bringe, um große Mengen der aufgeschlossenen Banken zu erzeugen, die von abgespaltenen Produkten befreit sind, anschließend jede dieser Banken für sich durch das entsprechende Enzym an der in dem Vektor vorhandenen Subklonierungsstelle zu spalten, jedes der Digestionsprodukte getrennt in einer Agarosegelschale anzuordnen, die Digestionsprodukte migrieren zu lassen, diese auf eine Membran zu transferieren und sie mit einer für das zu klonierende Gen spezifischen Sonde durch Homologie zu hybridisieren, um ihre CEM zu bestimmen,
- den Schritt (d),
- den Schritt (e).

11. Verwendung des Verfahrens nach Anspruch 9 zur Identifizierung
- von Allelen aus unterschiedlichen Quellen von Tieren derselben Spezies, oder, im Falle des Menschen, von unterschiedlichen Individuen,
- von genetischen Übereinstimmungen, die bei unterschiedlichen Spezies vorhanden sind,
- von alternativen Spleißungen eines Gens.
- von unterschiedlichen Mitgliedern einer genetischen Familie.

12. Southern-Blot-Verfahren zur Identifizierung von Inserts, wobei das Verfahren ein Klonierungsverfahren einer Nucleinsäuresequenz nach einem der Ansprüche 1 bis 9 verwendet, **dadurch gekennzeichnet, dass** das Verfahren umfasst:
- den Schritt (a),
- den Schritt (b),
- den Schritt (b'), zu dem es gehört, die in dem oben erwähnten Schritt (b) gewonnenen monoaufgeschlossenen Banken in kompetente Bakterien, oder äquivalente Wirte umzuwandeln,
- den Schritt (c), zu dem es gehört die Bakterien mit einem selektiven Milieu zusammen zu bringen, um aufgeschlossene Banken zu erzeugen, die von abgespaltenen Produkten befreit sind, anschließend jede dieser Banken für sich durch das bzw. die Enzyme zu spalten, die der (den) Stelle(n) der Subklonierung entsprechen, die die eine (oder mehreren) Konstruktionsstellen der Bank flankieren, und die Digestionsprodukte in Agarose- oder Acrylamidgelschalen anzuordnen, dieses Gel migrieren zu lassen und auf eine Membran zu übertragen und mit den verwendeten Identifizierungsinserten als markierte Sonde, sei es einzeln, sei es in Gruppen, zu hybridisieren, um ihre CEM zu bestimmen,

13. Verfahren zur Untersuchung des Polymorphismus, wobei das Verfahren ein Southern-Blot-Verfahren zur Identifizierung von Inserts nach Anspruch 12 verwendet, **dadurch gekennzeichnet, dass**:
- die genomische DNA-Bank des Schritts (a) wird zum Gegenstand der Untersuchung,
- die in Schritt (c) als Sonden verwendeten Inserte sind Marker für den Polymorphismus.

14. Verfahren zur Untersuchung des Polymorphismus, **dadurch gekennzeichnet, dass** dieses umfasst:
- die Schritte (a) bis (c) des Verfahrens nach einem der Ansprüche 1 bis 10, zu dem es gehört, die CEM jedes bekannten Markers und anschließend den Aufbau einer genomischen DNA-Bank zu definieren,
- den Schritt (d), zu dem es gehört, die Digestion durch Batterien von Enzymen zu bewirken, die der CEM entsprechen und den untersuchten Markern zugeordnet sind,
- den Schritt (d'), zu dem es gehört, die mehrfach aufgeschlossenen Banken in kompetente Bakterien umzuwandeln,
- den Schritt (e), zu dem es gehört, diese Bakterien in einem flüssigen oder festen Milieu zu züchten, das eine Substanz enthält, die für das Plasmid selektiv ist.

## Claims

1. Method for cloning a nucleic acid fragment, **characterized in that** it comprises the following steps:
a) a DNA library that may contain said nucleic acid fragment to be cloned is prepared by inserting DNA fragments of a sample into a vector devoid of any site for cleavage by restriction enzymes whose site comprises from 5 to 6 nucleotides corresponding to those used for the digestions of the library, but conserving at least one or two sites for the construction of the library.
b) The library is digested in parallel with at least 10 restriction enzymes whose site comprises from 5 to 6 nucleotides, so as to obtain as many single-digested libraries as there are enzymes used.
c) Each of the single-digested libraries obtained in the preceding step is tested so as to evaluate the integrity of the nucleic acid fragment to be cloned and thus establish its multiple enzymatic characteristic (MEC).
d) The complete initial library is taken up and digested simultaneously with the restriction enzymes that do not affect the integrity of the nucleic acid fragment to be cloned, so as to obtain a multidigested library.
e) The resistant clone containing the nucleic acid fragment to be cloned is isolated.

2. Method for cloning a nucleic acid fragment according to Claim 1, **characterized in that**, in step b), the library is digested with 50 to 70 restriction enzymes.

3. Method for cloning a nucleic acid fragment according to either of Claims 1 and 2, **characterized in that** the cloning vector of step (a) also comprises at least two other sites, that are identical or nonidentical, and different from the first site(s) and flanking it (them), and that are useful for subcloning the nucleic acid fragment once the latter has been isolated.

4. Method for cloning a nucleic acid fragment according to one of Claims 1 to 3, **characterized in that** it comprises, between steps (b) and (c), the following step:
b') the single-digested libraries are transfected independently into cellular hosts so as to obtain corresponding batches of cellular hosts.

5. Method according to any one of the preceding claims, **characterized in that** it comprises, between steps (a) and (b), the following step:
a') the presence of the nucleic acid fragment to be cloned in the library is verified by transfecting into a cellular host that does not have said fragment and testing the presence of the fragment in said host.

6. Method according to any one of the preceding claims, **characterized in that** it comprises, between steps (d) and (e), the following step:
d') competent hosts are transformed with the multidigested library of step (d), so as to verify the nature of the fragments cloned.

7. Method according to any one of Claims 1 to 6, **characterized in that** the DNA library contains from 1 to 10⁸ different fragments, each of the order of 0.1 kb to 5 kb.

8. Method according to any one of the preceding claims, **characterized in that** the tests carried out in steps (c) and/or (a') so as to verify the integrity of the nucleic acid sequence to be cloned are any means for demonstrating either the sequence itself, for instance a probe, or the protein encoded by said sequence, such as a ligand, for instance an antibody, or else the activity of this protein, for instance an enzymatic label, which can be detected by any means known to those skilled in the art, for instance fluorescent or radioactive labelling.

9. Method for cloning a gene by means of an expression library according to any one of Claims 1 to 8, **characterized in that** it comprises:
- step (a),
- step (a'), consisting in verifying the presence of the gene being sought in the library by transfecting into a cell line that does not have the activity or the phenotype being sought, and measuring its restoration so as to distinguish the transfected cells from the nontransfected cells,
- step (b),
- step (b'), consisting in independently transfecting the monodigested libraries of step (b),
- step (c), consisting in testing each of the batches obtained in step (b') for the presence of the activity associated with the gene to be cloned, and evaluating the integrity of the sequence of said gene in order to establish the MEC of the activity associated with said gene,
- step (d),
- step (d'), consisting in transforming competent bacteria with the multidigested library,
- step (e).

10. Method for homology-based cloning according to any one of Claims 1 to 8, **characterized in that** it comprises:
- step (a),
- step (b),
- step (b'), consisting in transforming competent bacteria with the digestion products of step (b),
- step (c), consisting in growing the transformed bacteria in a selective medium so as to produce large amounts of the digested libraries freed of the cleaved products, and then in separately cleaving each or these libraries with the enzyme corresponding to the subcloning site present in the vector, in separately depositing each of the digestion products in a well of an agarose gel, in migrating the digestion products, in transferring them onto a membrane and in hybridizing them with a probe specific for the gene to be cloned based on homology, in order to establish its MEC,
- step (d),
- step (e).

11. Application of the method according to Claim 9 to the identification:
- of alleles of various strains of animals of the same species, or of various individuals in humans,
- of gene equivalents present in various species,
- of alternative splicings of a gene,
- of various members of a gene family.

12. Southern blotting method for identifying inserts using a method for cloning a nucleic acid sequence according to any one of Claims 1 to 9, **characterized in that** it comprises:
- step (a),
- step (b),
- step (b'), consisting in transforming competent bacteria, or equivalent hosts, with the single-digested libraries obtained in step (b) above,
- step (c), consisting in growing the bacteria in a selective medium so as to produce digested libraries freed of the cleaved products, and then in separately cleaving each of these libraries with the enzyme(s) corresponding to the subcloning site(s) flanking the site(s) for the construction of the library, and in depositing the digestion products in the wells of an agarose or acrylamide gel, in carrying out migration of this gel and in transferring it onto a membrane, and in hybridizing them with the inserts to be identified, used as a labelled probe, either one by one or several at a time, in order to establish their MEC.

13. Method for studying polymorphism using the Southern blotting method for identifying inserts according to Claim 12, **characterized in that**:
- the genomic DNA library of step (a) is derived from the individual studied.
- The inserts used as probes in step (c) are polymorphism markers.

14. Method for studying polymorphism, **characterized in that** it comprises:
- steps (a) to (c) of the method according to one of Claims 1 to 10, consisting in defining the MEC of each of the known markers, and then in constituting a genomic DNA library,
- step (d), consisting in carrying out the digestion with batteries of enzymes corresponding to the MECs attributed to the markers studied,
- step (d'), consisting in transforming competent bacteria with the multidigested libraries,
- step (e), consisting in culturing these bacteria in a liquid or solid medium containing a selective agent for the plasmid.
